# EUROPEAN PATENT SPECIFICATION

(11) **EP 2 303 866 B1**
(45) Date of publication and mention of the grant of the patent: **02.08.2017**
(21) Application number: 09779911.8
(22) Date of filing: 23.06.2009
(51) Int. Cl.: C07D 401/12, A61K 31/4439

(54) **CRYSTALLINE FORMS OF RABEPRAZOLE SODIUM**
KRISTALLINE FORMEN VON RABEPRAZOL-NATRIUM
NOUVELLES FORMES CRISTALLINES DU RABÉPRAZOLE SODIQUE

(30) Priority: 23.06.2008 SI 200800161
(43) Date of publication of application: 06.04.2011
(73) Proprietor: KRKA, tovarna zdravil, d.d., Novo mesto, 8501 Novo mesto (SI)
(72) Inventor: KOTAR-JORDAN, Berta, 8311 Kostanjevica na Krki (SI); VRECER, Franc, 8531 Straza pri Novem mestu (SI); ZADNIK, Jernej, 8350 Dolenjske Toplice (SI); URSKA, Turk, 8000 Novo Mesto (SI); KOVACIC, Borut, 1251 Moravce (SI)
(74) Representative: Uexküll & Stolberg
(86) International application number: PCT/EP2009/057846
(87) International publication number: WO 2010/006904

(56) References cited:
- EP-A- 0 268 956
- EP-A- 0 342 522
- EP-A- 0 446 961
- EP-A- 1 452 533
- EP-A- 1 674 463
- EP-A- 1 681 056
- WO-A-03/082858
- WO-A-2005/092297
- WO-A-2007/070164
- WO-A-2007/091276
- JP-A- 2001 039 975
- US-B1- 6 296 875
- CAIRA M R: "CRYSTALLINE POLYMORPHISM OF ORGANIC COMPOUNDS" 1 January 1998 (1998-01-01), TOPICS IN CURRENT CHEMISTRY, SPRINGER, BERLIN, DE, PAGE(S) 163 - 208 , XP001156954 the whole document

## Description

### FIELD OF THE INVENTION

The present invention relates to a crystalline form of rabeprazole sodium, a process for its preparation and its uses. The invention also relates to pharmaceutical compositions comprising said"crystalline form of rabeprazole sodium.

### BACKGROUND OF THE INVENTION

Rabeprazole is used to treat conditions where the stomach produces too much acid, including ulcers, gastroesophageal reflux disease (GERD) and Zollinger-Ellison syndrome. Rabeprazole is used in combination with other medications to eliminate *H. pylori,* a bacteria that causes ulcers. Rabeprazole is in a class of medications called proton-pump inhibitors. It works by decreasing the amount of acid secreted by the parietal cells of the stomach.

Rabeprazole and its sodium salt were first disclosed in EP268956. Crystalline forms of rabeprazole sodium are described in JP2001039975, WO03082858, EP1452533, EP1674463, WO2007091276 and IP135066.

### BRIEF DESCRIPTION OF THE DRAWINGS

Figure 1 is an X-ray powder diffraction pattern of crystalline form F of rabeprazole sodium prepared according to Example 1.

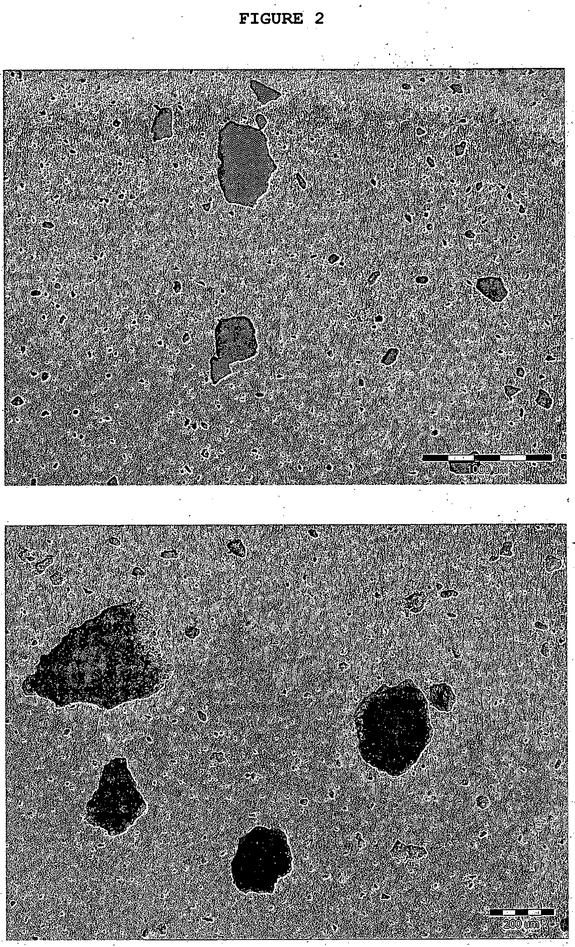

Figure 2 are pictures of particles of crystalline form F of rabeprazole sodium prepared according to Example 1.

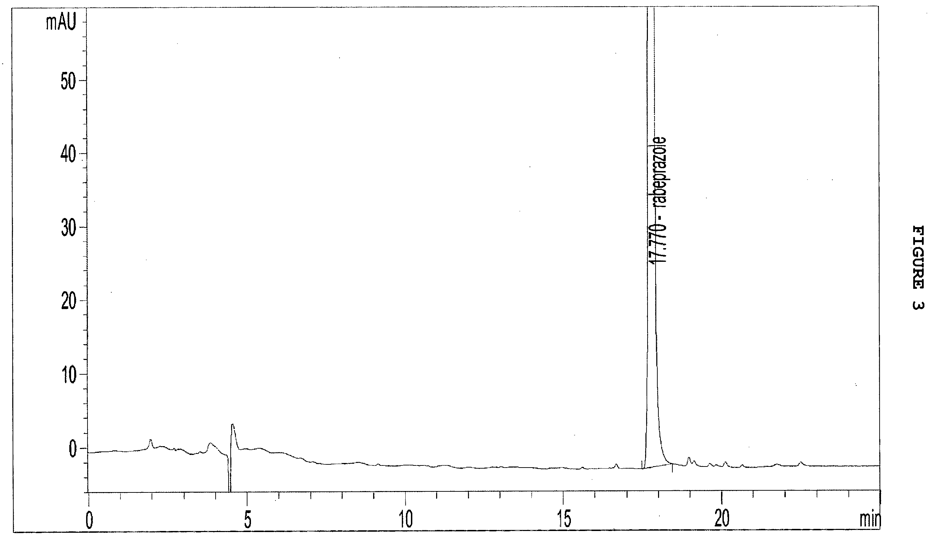

Figure 3 is a HPLC chromatogram of rabeprazole sodium prepared according to Example 1.

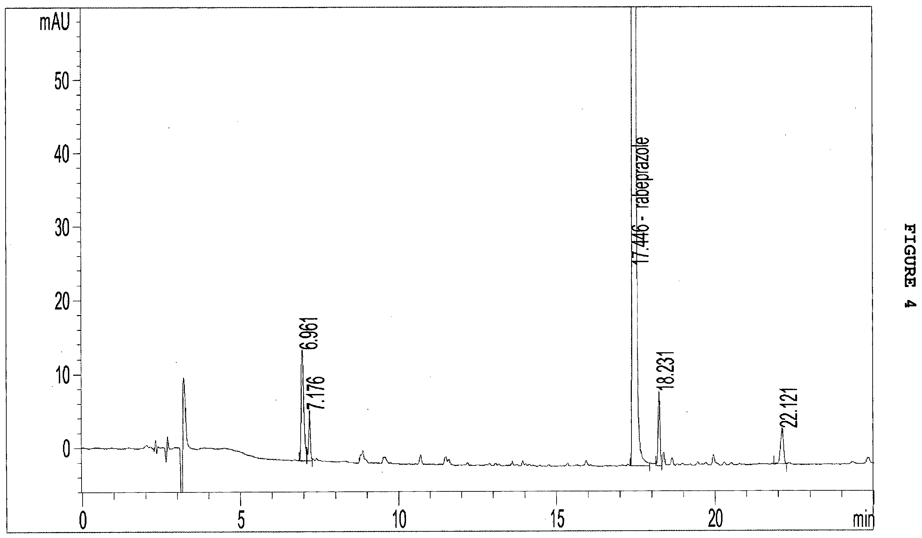

Figure 4 is a HPLC chromatogram of rabeprazole sodium tablet cores prepared according to Example 13.
Figure 5 is an X-ray powder diffraction pattern of crystalline form H of rabeprazole sodium prepared according to Example 6.

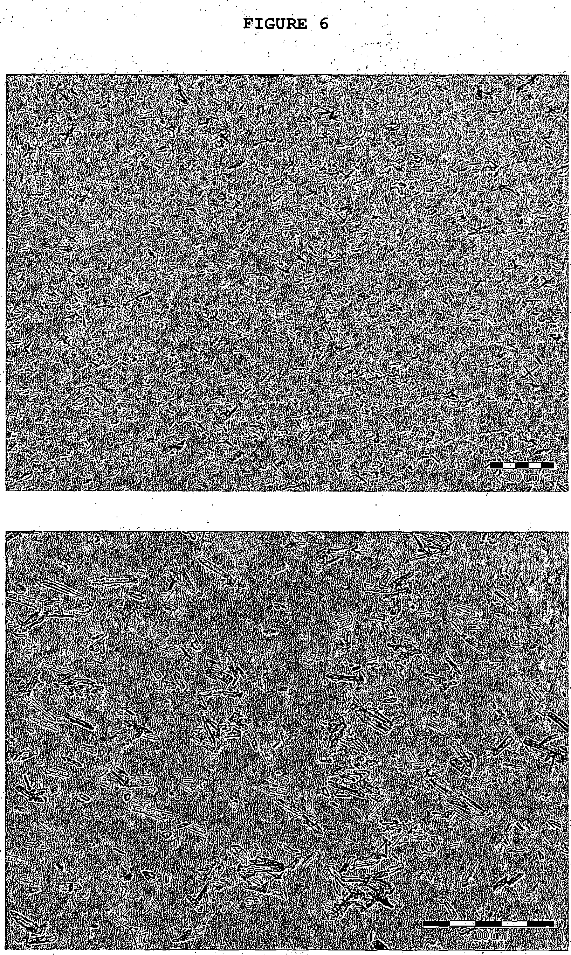

Figure 6 are pictures of particles of crystalline form H of rabeprazole sodium prepared according to Example 6.
Figure 7 is an X-ray powder diffraction pattern of crystalline form I of rabeprazole sodium prepared according to Example 7.

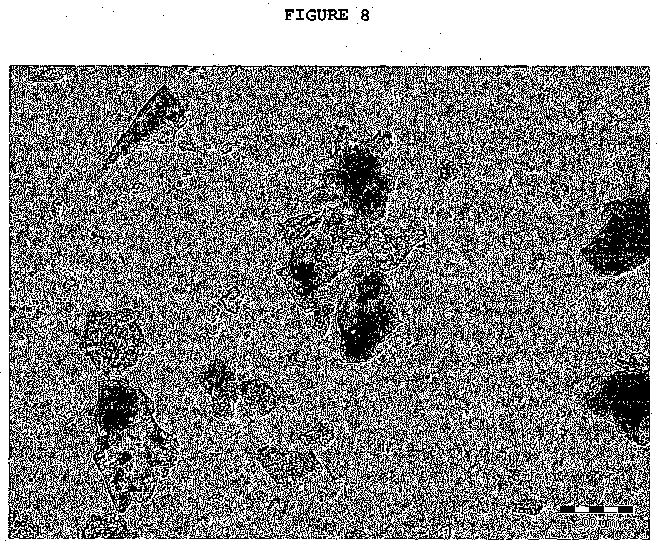

Figure 8 are pictures of particles of crystalline form I of rabeprazole sodium prepared according to Example 7.

X-ray powder diffraction patterns, images of particles and HPLC chromatograms were obtained as described in the examples.

### DESCRIPTION OF THE INVENTION

The present invention relates to a new crystalline form of rabeprazole sodium, which is suitable for the preparation of pharmaceutical dosage forms and other crystalline or amorphous forms of rabeprazole sodium.

More specifically, the present invention relates to crystalline form of rabeprazole sodium designated as form F and its use for the preparation of crystalline form of rabeprazole sodium designated as forms H and I. They may either be more than 90%, 95%, 99%, 99.5% or 99.9% of polymorphic purity or include other polymorphic forms, or an amorphous form.

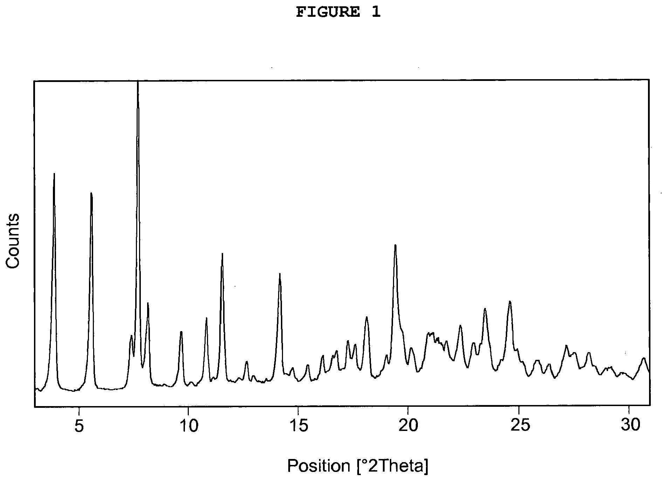

Crystalline form F of rabeprazole sodium prepared according to present invention is characterized by an X-ray powder diffraction pattern having peaks at about 3.9, 5.6, 7.7, 11.6, 14.2 ± 0.2 degrees two-theta. Crystalline form F of rabeprazole sodium can be further characterized by X-ray powder diffraction peaks at about 3.9, 5.6, 7.7, 11.6, 14.2, 19.5, 24.6 ± 0.2 degrees two-theta. The X-ray powder diffraction pattern of crystalline form F of rabeprazole sodium is shown in Figure 1.

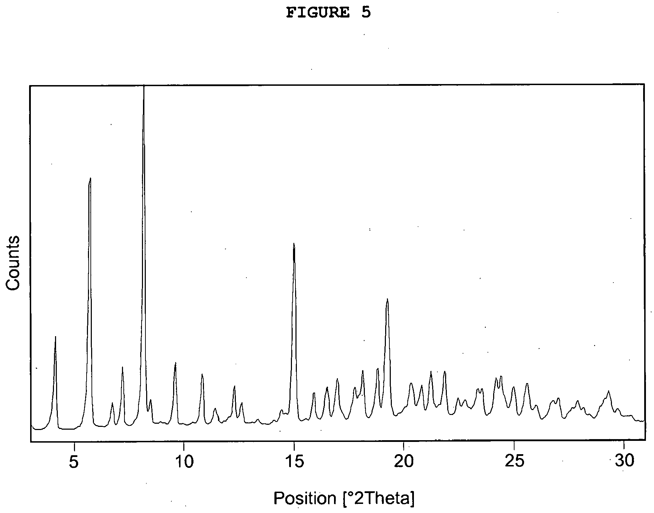

Crystalline form H of rabeprazole sodium prepared according to present invention is characterized by an X-ray powder diffraction pattern having peaks at about 4.1, 5.7, 8.2, 9.6, 15.0 ± 0.2 degrees two-theta. Crystalline form H of rabeprazole sodium can be further characterized by X-ray powder diffraction peaks at about 4.1, 5.7, 7.2, 8.2, 9.6, 15.0, 19.3 ± 0.2 degrees two-theta. The X-ray powder diffraction pattern of crystalline form H of rabeprazole sodium is shown in Figure 5.

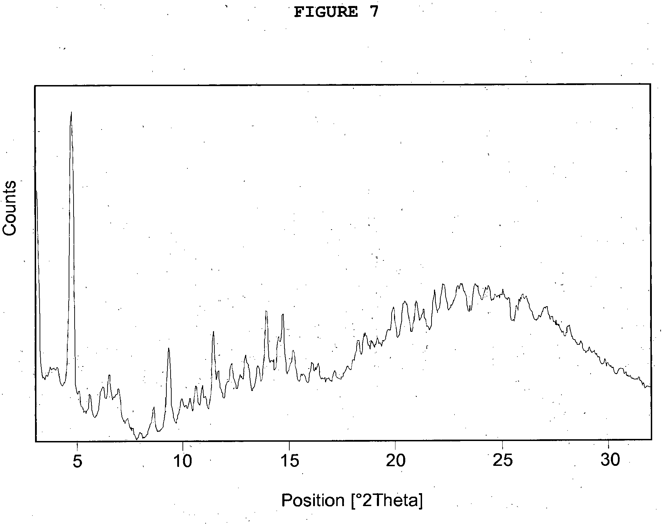

Crystalline form I of rabeprazole sodium prepared according to present invention is characterized by an X-ray powder diffraction pattern having peaks at about 4.8, 9.3, 11.5, 13.9, 14.7 ± 0.2 degrees two-theta. The X-ray powder diffraction pattern of crystalline form I of rabeprazole sodium is shown in Figure 7.

X-ray powder diffraction patterns can be obtained in particular as described in the examples.

In a further aspect, the present invention provides a process for the preparation of crystalline form F.

The process for preparation of crystalline form F of rabeprazole sodium comprises the steps of:
a) preparing a solution of rabeprazole sodium in a nitrile solvent with less than 10% molar excess, particularly less than 5% molar excess, more preferably from 1 to 5% molar excess, most preferably from 2 to 4% molar excess of sodium cations in regard to rabeprazole;
b) crystallizing rabeprazole sodium from the solution by adding an antisolvent; and
c) isolating the resulting precipitate.

Preferably, the nitrile solvent for preparation of crystalline form F of rabeprazole sodium is acetonitrile. The antisolvent is preferably an ether, more preferably tert-butyl methyl ether (TBME) or diisopropyl ether. It is further preferred that the nitrile solvent and the antisolvent are used in a ratio of 5:1 to 1:5, particularly 2:1 to 1:3, more preferably 1:1 to 1:2. In particular, the concentration of rabeprazole sodium at the beginning of crystallization is less than 0.3 g/ml, particularly between 0.01 and 0.3 g/ml, more preferably less than 0.2 g/ml, most preferably between 0.05 and 0.15 g/ml.

Step b) can be conducted by slowly adding the antisolvent to the solution of rabeprazole sodium in the nitrile solvent or vice versa, preferably at a temperature of 0 to 50 °C, particularly 10 to 30 °C, more preferably 15 to 25°C, most preferably 20 to 25°C. The solution can be maintained at said temperature or can be cooled to a temperature of 0 to 15°C, more preferably 5 to 15°C, most preferably 8 to 12°C to begin crystallization.

A process for preparation of crystalline form H of rabeprazole sodium comprises the step of drying rabeprazole sodium form F at a temperature of not less than 60°C and relative humidity of not more than 30%.

A process for preparation of crystalline form I of rabeprazole sodium comprises the step of leaving the rabeprazole sodium form F to stand at a temperature of not more than 40°C and at relative humidity of at least 60% for at least five hours, preferably at a temperature of not more than 30°C and at relative humidity of at least 70%, most preferably at a temperature of not more than 25°C and at relative humidity of at least 70%.

Rabeprazole used in the present invention can be prepared according to already known methods such as methods disclosed in EP268956, WO2004063188, WO2006024890, WO2006117802, EP1818331, WO2008017020, IP135066, IP127347, WO0168594, EP1575935 or WO0179194. It can also be further recrystallized and/or purified to a purity level more than 99%, 99.5% or 99.9 %. If the crystalline forms F, H or I of rabeprazole sodium prepared by the present invention are not satisfactorily pure, they can be recrystallized to a purity level more than 99%, 99.5% or 99.9 %.

The average particle size of crystalline forms F, H or I of rabeprazole sodium prepared according to the present invention can be in the range of 0.1-600 µm. Average particle sizes can be determined in particular as described in the examples. If smaller particles are needed, the obtained crystalline form of rabeprazole sodium can be sieved, milled or micronized optionally together with other excipients. In the case of agglomerates ultrasonication can be used.

Crystalline forms F, H or I of rabeprazole sodium can be used for preparation of other crystalline or amorphous forms of rabeprazole or rabeprazole sodium, such as crystalline form Z (EP1452533, IP135066), II (JP200139975), X and Y (WO03082858) or amorphous form, which can be further used for preparation of pharmaceutical composition.

The present invention also relates to a pharmaceutical composition prepared from crystalline form F of rabeprazole sodium as active ingredient in admixture with one or more carriers or auxiliary substance(s) conventionally applied in the pharmaceutical industry.

In particular, the present invention also relates to a pharmaceutical composition comprising crystalline form F of rabeprazole sodium in combination with at least one pharmaceutically acceptable carrier or auxiliary substance.

The water content in crystalline forms F, H or I of rabeprazole sodium used in the preparation of pharmaceutical compositions according to present invention is preferably below 8%, more preferably below 6% and most preferably below 4%.

Crystalline forms F, H or I of rabeprazole sodium can be formulated in solid dosage forms such as tablets, pellets, capsules designated as drug containing core coated by at least one film coating insoluble in media with pH below 4.5.

Drug containing core can be manufactured by the state of the art processes such as direct compression, granulation, pelletization.

In one embodiment of the present invention rabeprazole sodium containing core is produced by direct compression of powder mixture of rabeprazole sodium and at least one further ingredient selected from stabilizers, diluents, binders, disintegrants, glidants and lubricants, where stabilizers used in the ratio to drug from 1:5 to 5:1 can be selected from water insoluble alkaline reacting compounds having pH of saturated solution of such alkaline reacting substance in purified water at least 8.0, preferably 8.5 and most preferably 9.0 such as alkali or earth alkali oxides, hydroxides, carbonates, phosphates or mixed inorganic salts or water soluble alkaline reacting compounds such as alkali hydroxides, carbonates, di- and tribasic phosphates (e.g. disodium hydrogen phosphate, trisodium phosphate) and organic bases such as meglumine or triethanolamine.

Diluents can be selected from sucrose, lactose, mannitol, dextrose, sorbitol, trehalose, starch and its derivatives, microcrystalline cellulose. Diluents are preferably used in the range 10 - 60 % (w/w), more preferably in the range 15 - 50 %. Mannitol for direct compression is preferred.

Binders can be selected from water soluble polymers such as soluble cellulose ethers such as hydroxypropylmethyl cellulose (having viscosity of 2 % solution in water at 20°C below 20 cps (determined according to USP method), preferably below 15 cps and most preferably below 10 cps), hydroxypropyl cellulose, which contains not less than 52.0 % and not more than 81.0 % of hydroxypropoxy groups (-OCH₂CHOHCH₃), hydroxyethyl cellulose, methyl cellulose, povidone, copovidone, polyvinyl alcohol and/or water insoluble polymers such as starch and its derivatives and microcrystalline cellulose. Binders are preferably used up to 10 % (w/w), more preferably from 1 to 8 % (w/w) and most preferably from 2 to 6 % (w/w).

Disintegrants can be selected from crospovidone, sodium carboxymethyl cellulose, calcium carboxymethyl cellulose, sodium starch glycolate, low-substituted hydroxypropyl cellulose which contains not less than 5.0 0 % and not more than 16.0 % of hydroxypropoxy groups (-OCH₂CHOHCH₃), polacriline potassium. Low-substituted hydroxypropyl cellulose is preferred.

Glidants can be selected from colloidal silicon dioxide (Aerosil), talc, magnesium trisilicate, magnesium silicate, calcium phosphate, powdered cellulose and magnesium oxide.

Lubricants can be selected from metal stearates such as magnesium, calcium, zinc or aluminium stearate, sodium starch fumarate, hydrogenated vegetable oils and stearic acid.

In another embodiment of the invention rabeprazole sodium containing core can be produced via granulation, here the granulation process can be performed in the presence of solvent such as wet granulation processes in fluid bed or high shear granulators, where organic solvents or the mixtures thereof can be used as granulation liquid or in the absence of solvent such as dry granulation processes like roller compaction or slugging or melt granulation processes are employed. The granulate is formulated by transformation of powder mixture of rabeprazole sodium according to present invention, stabilizer and at least one further ingredient selected from diluents, binders, disintegrants, glidants, lubricants or surface active substances which can be the same as used in case of direct compression except for binders where beside binders such as water soluble polymers such as soluble cellulose ethers such as hydroxypropyl methylcellulose (having viscosity of 2 % solution in water at 20°C below 20 cps (determined according to USP method), preferably below 15 cps and most preferably below 10 cps), hydroxypropyl cellulose which contains not less than 52.0% and not more than 81.0 % of hydroxypropoxy groups (-OCH₂CHOHCH₃), hydroxyethyl cellulose, methyl cellulose, povidone, copovidone, polyvinylalcohol and/or water insoluble polymers such as starch and its derivatives, and microcrystalline cellulose, also binders with melting point below 75°C, preferably below 55°C and most preferably between 35 and 45°C such as poloxameres, polyethyleneglycoles with MW below 10,000, partial glycerides or sugar esters are incorporated into granules by the before mentioned state of the art processes such as dry, wet or melt granulation. Surface active substances can be selected from anionic surfactants such as sodium lauryl sulphate and or non-ionic surfactants such as polysorbates, sugar esters or poloxameres. The obtained granulate is mixed with extragranular excipients selected from stabilizers, diluents, disintegrants, glidants and lubricants and compressed into tablet cores. Examples of stabilizers are water insoluble alkaline reacting compounds having pH of saturated solution of such alkaline reacting substance in purified water at least 8.0, preferably 8.5 and most preferably 9.0 or water soluble alkaline reacting compounds. Examples of alkaline reacting compounds are alkali or earth alkali oxides, hydroxides, carbonates, phosphates, mixed inorganic salts such as magnesium hydroxide carbonate, alkali metal hydroxides such as sodium or potassium hydroxide, alkali carbonates, alkali di- or tribasic phosphates such as disodium hydrogen phosphate or trisodium phosphate or composite substances such as Al₂O₃.6MgO.CO₂.12H₂O or similar compounds, organic bases such as meglumine, triethanolamine, diethanoleamine, trihidroxymethylaminomethane, salts of week organic acids such as potassium or sodium citrate, sodium carbonate. Examples of disintegrants are crospovidone, sodium carboxymethyl cellulose, calcium carboxymethyl cellulose, sodium starch glycolate, low-substituted hydroxypropyl cellulose having not less than 5.0% and not more than 16.0% of hydroxypropoxy groups (-OCH₂CHOHCH₃). Glidants and lubricants can be selected form colloidal silicon dioxide (Aerosil), metal stearates such as magnesium, calcium, zinc or aluminium stearate, sodium starch fumarate, hydrogenated vegetable oils, and stearic acid.

In a special embodiment of the invention drug containing core can be in the form of pellets obtained by the state of the art processes such as direct pelletization by extrusion and spheronization, pelletization in rotor processors or high shear granulators or pelletization by layering of inactive seeds such as neutral pellets made of sucrose and maize starch or microcrystalline cellulose with a powder or suspension coating with rabeprazole sodium admixed with stabilizer and at least one further excipient selected from binders, diluents, surface active substances, antitacking agents and/or other suitable ingredients. Water or water miscible solvents such as ethanol, methanol, acetone or mixture thereof can be used as solvents for coating.

Drug containing core can optionally be coated with separating coating with the thickness of at least 5µm, preferably at least 10 µm composed of polymer forming a low permeable film for water vapour such as ethylcellulose or polyvinylalcohol and optionally at least one excipient selected from stabilizers, water soluble additives such as mannitol, lactose, polyethyleneglycole and/or antitacking agents. In a special embodiment of the invention drug containing core can optionally be coated with water soluble coatings based on water soluble polymers selected from cellulose ethers such as low viscosity grades of hydroxypropylmethyl cellulose, hydroxypropyl cellulose, hydroxyethyl cellulose, methyl cellulose. Such coating can optionally contain further ingredients such as stabilizers, antitacking agents, plasticizers, pigments and colorants.

Drug containing core optionally coated with separating layer is further coated with a coating insoluble in acids preferably in acidic solutions with pH below 4.5. Coating insoluble in acids is formulated from polymers insoluble in acids such as hypromellose phthalate, polyvinyl acetate phthalate, methacrylic acid copolymers, cellulose acetate phthalate, hydroxypropylmethyl cellulose acetate succinate, and optionally other excipients selected from plasticizers, antitacking agents, pH modifiers. The pH of dispersion for coating of drug containing cores with a coating insoluble in acids (gastro resistant coating) can optionally be adjusted to pH 4-5.8 by partial neutralization of polymers with pH modifiers such as alkali hydroxides or ammonia. The thickness of acid insoluble coating is in the range 30-150 µm, preferably 40-130 µm and most preferably 50-120 µm.

The obtained solid dosage form coated with acid insoluble coating can optionally be further coated with an overcoat soluble in water and diluted acids improving the mechanical resistance, visual appearance and or decreasing the permeability for gases such as water vapour or oxygen of the acid insoluble coating.

Humidity and water content is a highly important parameter to control in order to achieve a stable final solid dosage formulation. Consequently, the water content in the solid dosage form should be limited to be below 2 % w/w determined by pharmacopoeia Karl-Fisher method. Besides, the primary packaging material should therefore protect the formulation from the ambient humidity. Tablets containing rabeprazole sodium can thus be packed in air tight containers such as HDPE containers with closure such as PP closure and with or without desiccator, aluminium foil blisters or Aclar® blisters or any other suitable water vapour impermeable packaging. Additionally inert gases such as nitrogen, argon or helium can be used to assure inert atmosphere in the formulation environment in the sealed packaging.

We have unexpectedly found that the particle size of water insoluble alkaline stabilizer and its surface area have profound effect on the stability of active pharmaceutical ingredient of benzimidazole type in the pharmaceutical composition. We have found that alkaline stabilizers with small particles and/or big surface area give much better stability.

Also disclosed is a pharmaceutical composition which comprises:
a) acid sensitive benzimidazole derivative or its pharmaceutically acceptable salt;
b) alkaline stabilizer with an average particle size below 75 µm or/and specific surface area of more than 0.5 m²/g.

Average particle sizes and specific surface areas can be determined in particular as described in the examples.

Preferably, the acid sensitive benzimidazole derivative is a proton pump inhibitor such as rabeprazole, omeprazole, lansoprazole, pantoprazole, their enantiomers or their pharmaceutically acceptable salts. The alkaline stabilizer is preferably an inorganic alkaline salt. More preferably, the alkaline stabilizer is selected from the group consisting of magnesium or calcium oxide, magnesium hydroxide carbonate, magnesium or calcium carbonate, magnesium or calcium phosphate. Most preferably, the acid sensitive benzimidazole derivative is rabeprazole sodium and the alkaline stabilizer is magnesium oxide.

Particle size of alkaline stabilizer is below 75 µm and/or specific surface area is more than 0.5 m²/g. Preferably, the particle size of alkaline stabilizer is below 50 µm and/or specific surface area is more than 2.0 m²/g. Most preferably, the particle size of alkaline stabilizer is below 25 µm and/or specific surface area is more than 4.0 m²/g.

The invention is illustrated by reference to the following examples.

### EXAMPLES

X-ray powder diffraction patterns were obtained by Philips PW3040/60 X'Pert powder diffractometer, X'celerator detector at CuKα radiation, 1.54178 Å, 3°<2θ<30°.

Images of particles were taken on a Microscope Olympus BX 50 equipped with Olympus camera DP70. Sample was prepared as suspension in paraffin oil.

The average particle size of rabeprazole sodium particles was determined by laser diffraction using a Malvern Mastersizer 2000 laser diffraction instrument with silicon fluid F10 as the dilution medium.

Specific surface area of MgO (magnesium oxide) was measured by gas sorption system Tristar 3000 based on nitrogen adsorption, using 6 point Brunauer, Emmett and Teller (BET) method with prior degassing of the sample at room temperature.

The average particle size of MgO was determined by laser diffraction using a Malvern Mastersizer 2000 laser diffraction instrument equipped with dry powder feeder Scirocco. The analysis was performed by dispersing a weighed amount of MgO in the air stream.

HPLC chromatogram of rabeprazole sodium was obtained on a HP 1100 chromatograph with DAD detector. Chromatographic separation was achieved using LUNA PFP 150X4.6 mm, 5 µm analytical column. Chromatographic conditions used were: sample concentration of 0.4 mg/mL, detection at 285 nm, flow of 0.6 mL/min, volume of injection 5 µL. A gradient elution using mobile phase A (0,01 M ammonium acetate pH 7.5 with 25% ammonium solution) and mobile phase B (CH₃CN) was employed with time table (0 min 7% B, 17 min 50% B, 22 min 50% B, 28 min 7% B), run time 35 min.

Solution for HPLC analysis of rabeprazole sodium was prepared by dissolving 20 mg of rabeprazole sodium in 50 ml of 0.05 M NaOH(aq):acetonitrile, 70:30 (V/V).

HPLC chromatogram of rabeprazole sodium tablet cores was obtained on a HP 1100 chromatograph with DAD detector. Chromatographic separation was achieved using Eclipse Plus C18 150x4.6 mm 1.8 µm analytical column. Chromatographic conditions used were: sample concentration of 0.4 mg/ml, detection at 285nm, flow of 0.6ml/min, volume of injection 5 µL. A gradient elution using mobile phase A (0,01 M ammonium acetate pH 7.5 with 25% ammonium solution) and mobile phase B (CH₃CN) was employed with time table (0 min 7% B, 17 min 50% B, 22 min 50% B, 28 min 7% B), run time 35 min.

Solution for HPLC analysis of tablet cores was prepared by dissolving weighted amount of tablet core powder corresponding to 20 mg of rabeprazole sodium in 50 ml of 0.05 M NaOH(aq): acetonitrile, 70:30 (V/V).

### I. SYNTHESIS

### Example 1: Rabeprazole sodium form F

20 g of rabeprazole sodium as amorphous form was dissolved in 100 ml of acetonitrile with addition of 0.2 ml 30% aqueous sodium hydroxide at 20-25°C. The solution was gradually dropped to 200 ml diisopropyl ether at 20-25°C. The oily solution was maintained at the same temperature to begin crystallization and aged for 16 hours. The product was filtered, washed with acetonitrile and once again slurred in 50 ml of acetonitrile for one hour. Filtered product was dried in vacuo at 40 °C to obtain 13.0 g of crystalline form F.

### Example 2: Rabeprazole sodium form F

20 g of rabeprazole sodium as amorphous form was dissolved in 100 ml of acetonitrile with addition of 0.2 ml 30% aqueous sodium hydroxide at 20-25°C. The solution was gradually dropped to 200 ml t-butyl methyl ether at 20-25°C. The oily solution was cooled to 10°C to begin crystallization and aged for 20 hours at 20 -25°C. The product was filtered, washed with acetonitrile and dried in vacuo at 40 °C to obtain 13.7 g of crystalline form F.

### Example 3: Preparation of rabeprazole sodium form Z from crystalline form F

30.0 g (0.0786 moles) of rabeprazole sodium in crystalline form F was suspended in a mixture of 120 ml toluene and 1.35 g (0.0275 moles) of sodium methoxide in 5 ml methanol at 35-30°C. The colloidal solution was heated to the reflux temperature at which methanol was distilled off, also including toluene (together about 20 ml). Crystallization appeared at boiling temperature and the suspension was gradually cooled to 20-25 °C and maintained at this temperature for another two hours. The product was filtered, washed with toluene and dried in vacuo at 50°C to obtain 28.8 g of crystalline form Z.

### Example 4: Preparation of rabeprazole sodium mix form II & Z

5.0 g (13.9 mmoles) of rabeprazole was dissolved in a solution of 0.75 g (18.7 mmoles) sodium hydroxide in 10 ml methanol at room temperature. Any undissolved particles were filtered off to get a clear solution. The solution was concentrated to an oily residue, 10 ml of methylene chloride was added and evaporated to dryness. The obtained dry product was dissolved in 25 ml of i-butyl methyl ketone at room temperature, stirred for one day until crystallization occurred then cooled to 0-5°C for 2 hours. The product was filtered and dried in vacuo at 50°C to obtain 3.1 g of crystalline form II and Z.

### Example 5: Preparation of rabeprazole sodium amorphous form from crystalline forms F, H or I

12.0 g (31.4 mmoles) of rabeprazole sodium was dissolved in 25 ml of methanol previously treated with 0.61 g (11.0 mmoles) of sodium methoxide at room temperature. Any undissolved particles were filtered off to get a clear solution. The solution was concentrated to an oily residue, 20 ml of methylene chloride was added and evaporated to dryness. The obtained product was dried again in vacuo at 60°C to obtain 12.3 g (with assay of water 4.0%, KF).

1.0 g so prepared rabeprazole sodium was suspended in 10 ml of ether (diethyl ether or *t*-butyl methyl ether or diisopropyl ether) or alkanes (heptane or hexane) aged for two days at 20-25°C. The product was filtered and dried in vacuo at 50°C to obtain stable amorphous form:

| | |
|---|---|
| 0.96 g | (diethyl ether) |
| 0.68 g | (*t*-butyl methyl ether) |
| 0.77 g | (diisopropyl ether) |
| 0.96 g | (heptane) |
| 0.93 g | (hexane) |

### Example 6: Rabeprazole sodium form H

Rabeprazole sodium form F was dried in a dryer over P₂O₅ at the final temperature 80°C.

### Example 7: Rabeprazole sodium form I

Rabeprazole sodium form F was held at 25°C and at relative humidity 70% for 10 hours.

### Example 8: Synthesis and crystallization of rabeprazole sodium form F

137.20 g (0.400 moles) Rabeprazole sulphide was dissolved in 360 ml 2,2,2-trifluoroethanol and cooled to about -20°C. Then 34.3 ml of hydrogen peroxide 35% was added following with gradually addition of solution 200 mg methyltrioxorhenium(VII) in 40 ml of 2,2,2-trifluoroethanol as catalyst for the reaction. The catalyst was added in portions. The mixture was stirred for up to 0°C and the end of the reaction was determined upon in-process control compliance (HPLC assay for sulphide not more than 5 %).

After the reaction was completed 5 % aqueous solution of sodium thiosulphate were added at 10 -15°C and pH of the solution was adjusted to 11 with 10 % aqueous solution of sodium hydroxide. 2,2,2-Trifluoroethanol was distilled off. 600 ml of acetonitrile/water mixture (1:3, v/v) was slowly added to the concentrated residue at about 15°C and activated carbon was added to decolour the solution. The suspension was filtered through the randalite.

Rabeprazole was isolated by precipitation with 50 % acetic acid. pH was adjusted to about 8.5 at about 15°C to perform crystallization of rabeprazole, then cooled up to 0°C. The crystallised product was collected by filtration, washed or macerated with acetonitrile. The product was dried in dryer below 45°C, yield is 120 g.

120.0 g of rabeprazole was suspended in 600 ml of acetonitrile at room temperature. Sodium salt was prepared with 26.8 g 50 % aqueous solution of sodium hydroxide and heating up to 50°C to obtain a solution. Filtration was included to remove any undissolved particles. Rabeprazole sodium was precipitated with 600 ml diisopropyl ether which was slowly added to the solution at 20 ± 3 °C. The crystallization was carried out for up to 10 hours at 20 ± 3 °C, then gradually cooled up to 0 °C and left standing for about 5 hours. The crystallised product was collected by filtration and washed with diisopropyl ether. The product is dried in dryer below 45 °C. Yield is 122 g of rabeprazole sodium form F.

### II. PHARMACEUTICAL FORMULATIONS

Rabeprazole sodium used in the following examples can be in any crystalline or amorphous form, such as form F, H or I according to present invention or crystalline form II, X, Y, Z or amorphous form already known from prior art.

### Example 9: Direct compression

Rabeprazole sodium, mannitol (Parteck M200), magnesium oxide, hydroxypropyl cellulose (HPC Klucel EF), low-substituted hydroxypropyl cellulose (L-HPC LH-11) and magnesium stearate were sieved and mixed. The mixture was treated with a tablet machine to thereby give uncoated tablets of the following composition each weighing 150.0 mg

### Composition:

| Ingredient | mg/tablet core |
|---|---|
| Rabeprazole sodium | 20.00 |
| Mannitol | 52.00 |
| Magnesium oxide | 45.00 |
| Hydroxypropyl Cellulose, Low-substituted | 15.00 |
| Hydroxypropyl Cellulose | 15.00 |
| Magnesium stearate | 3.00 |

### Example 10: Direct compression

Rabeprazole sodium, mannitol (Parteck M200), magnesium oxide, hydroxypropyl cellulose (HPC Klucel EF), low-substituted hydroxypropyl cellulose (L-HPC LH-11) and magnesium stearate were sieved and mixed. The mixture was treated with a tablet machine to thereby give uncoated tablets of the following composition each weighing 150.0 mg

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 19.50 |
| Hydroxypropyl Cellulose | 10.50 |
| Magnesium stearate | 3.00 |

### Example 11: Direct compression

Rabeprazole sodium, mannitol (Parteck M200), magnesium oxide, hydroxypropyl cellulose (HPC Klucel EF), low-substituted hydroxypropyl cellulose (L-HPC LH-11) and magnesium stearate were sieved and mixed. The mixture was treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 22.50 |
| Hydroxypropyl Cellulose | 7.50 |
| Magnesium stearate | 3.00 |

### Example 12: Wet granulation

Rabeprazole sodium, mannitol (Parteck M200), magnesium oxide and hydroxypropyl cellulose (HPC Klucel EF) were sieved and mixed together. To the obtained mixture ethanol was added. The resulting mixture was granulated, dried and passed through an 18-mesh sieve to give dry granules, which were mixed with low-substituted hydroxypropyl cellulose (L-HPC LH-11) and magnesium stearate. The mixture was treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 15.00 |
| Hydroxypropyl Cellulose | 15.00 |
| Magnesium stearate | 3.00 |

### Example 13: Wet granulation

Rabeprazole sodium, mannitol (Parteck M200), magnesium oxide (Magnesia 22), hydroxypropyl cellulose (HPC Klucel EF), low-substituted hydroxypropyl cellulose (L-HPC LH-11) were sieved and mixed together. To the obtained mixture ethanol was added. The resulting mixture was granulated, dried and passed through an 18-mesh sieve to give dry granules, which were mixed with additional mannitol, additional low-substituted hydroxypropyl cellulose (L-HPC LH-11) and magnesium stearate. The mixture was treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 21.00 |
| Hydroxypropyl Cellulose | 9.00 |
| Magnesium stearate | 3.00 |

### Example 14: Wet granulation

Rabeprazole sodium, mannitol (Parteck M200), magnesium oxide (Magnesia 27), hydroxypropyl cellulose (HPC Klucel EF), low-substituted hydroxypropyl cellulose (L-HPC LH-11, L-HPC LH-21) were sieved and mixed together. To the obtained mixture methanol was added. The resulting mixture was granulated, dried and passed through an 18-mesh sieve to give dry granules, which were mixed with additional mannitol, additional low-substituted hydroxypropyl cellulose (L-HPC LH-11) and magnesium stearate. The mixture was treated with a tablet machine to thereby give uncoated tablets of the following composition each weighing 150.0 mg

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 21.00 |
| Hydroxypropyl Cellulose | 9.00 |
| Magnesium stearate | 3.00 |

### Example 15: Intermediate Coating

50 g of ethylcellulose (Ethocel 45cP) was dissolved in 500 g of ethanol and 50 g of magnesium oxide (Magnesia 27) was dispersed in the obtained solution. The uncoated tablets obtained in Example 14 were intermediately coated with the above dispersion. Thus intermediately coated tablets with an average weight 153 mg were obtained. Tablet cores prepared according to Examples 9-13 were coated with the same procedure.

### Example 16: Wet granulation

Rabeprazole sodium, mannitol (Parteck M200), magnesium oxide (Magnesia 27), hydroxypropyl cellulose (HPC Klucel EF), low-substituted hydroxypropyl cellulose (L-HPC LH-11) were sieved and mixed together. To the obtained mixture ethanol was added. The resulting mixture was granulated, dried and passed through an 18-mesh sieve to give dry granules (A), which were mixed with additional mannitol, additional low-substituted hydroxypropyl cellulose (L-HPC LH-11) and magnesium stearate. The mixture was treated with a tablet machine to thereby give uncoated tablets of the following composition each weighing 150.0 mg

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 21.00 |
| Hydroxypropyl Cellulose | 9.00 |
| Magnesium stearate | 3.00 |

### Example 17: Intermediate coating

50 g of ethylcellulose (Ethocel 20cP) was dissolved in 500 g of ethanol and 50 g of magnesium oxide (Magnesia 27) was dispersed in the obtained solution. The uncoated tablets obtained in Example 16 were intermediately coated with the above dispersion. Thus intermediately coated tablets with an average weight 152 mg were obtained.

### Example 18: Enteric coating

200 g of hydroxypropyl methylcellulose phthalate, 10 g of titanium oxide, 20 g of talc, 10 g of red iron oxide and 20 g of a glycerol fatty acid ester (Myvacet 9/08) were dissolved and/or dispersed in a mixture of 80% ethanol with water. The intermediately coated tablets obtained in Example 17 were then coated with a resulting solution in coating pan. Thus enteric tablets with an average weight 167 mg were obtained.

### Example 19: Fluid-bed granulation

Hydroxypropyl cellulose (HPC Klucel EF) was dispersed in the solution of rabeprazole sodium in ethanol. Obtained dispersion was sprayed onto mannitol particles in fluid bed granulating system to give granules. Dry granules, magnesium oxide, granulated magnesium oxide, low-substituted hydroxypropyl cellulose (L-HPC LH-11) and magnesium stearate were mixed together and treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.50 |
| Magnesium oxide | 12.00 |
| Magnesium oxide, granulated | 48.00 |
| Hydroxypropyl Cellulose, Low-substituted | 20.50 |
| Hydroxypropyl Cellulose | 9.00 |
| Magnesium stearate | 3.00 |

### Example 20: Fluid-bed granulation

Hydroxypropyl cellulose (HPC Klucel EF) and magnesium oxide (Magnesia 27) were dispersed in the solution of rabeprazole sodium in ethanol. Obtained dispersion was sprayed onto granulated magnesium oxide particles in fluid bed granulating system to give granules. Dry granules, mannitol (Parteck M200), magnesium oxide (Magnesia 27), low-substituted hydroxypropyl cellulose (L-HPC LH-11) and magnesium stearate were mixed together and treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.50 |
| Magnesium oxide | 12.00 |
| Magnesium oxide, granulated | 48.00 |
| Hydroxypropyl Cellulose, Low-substituted | 25.00 |
| Hydroxypropyl Cellulose | 4.50 |
| Magnesium stearate | 3.00 |

### Example 21: Intermediate coating

40 g of ethylcellulose (Ethocel 20cP) was dissolved in 580 g of ethanol and 40 g of magnesium oxide (Magnesia 27) was dispersed in the obtained solution. The uncoated tablets obtained in Example 20 were intermediately coated with the above dispersion. Thus intermediately coated tablets with an average weight 152 mg were obtained. Tablet cores prepared according to Examples 19 were coated with the same procedure.

### Example 22: Intermediate coating

40 g of ethylcellulose (Ethocel 20cP) was dissolved in 580 g of ethanol and 60 g of magnesium oxide (Magnesia 27) is dispersed in the obtained solution. The uncoated tablets obtained in Example 20 were intermediately coated with the above dispersion. Thus intermediately coated tablets with an average weight 152 mg were obtained. Tablet cores prepared according to Example 19 were coated with the same procedure.

### Example 23: Enteric coating

200 g of hydroxypropyl methylcellulose phthalate, 10 g of titanium oxide, 20 g of talc, **6** g of red iron oxide and 20 g of a glycerol fatty acid ester (Myvacet 9/08) were dissolved and/or dispersed in a mixture of 80% ethanol with water. The intermediately coated tablets obtained in Examples 21 and 22 were then coated with a resulting solution in coating pan.

### Example 24: Enteric coating

200 g of hydroxypropyl methylcellulose phthalate, 10 g of titanium oxide, 20 g of talc, **3** g of red iron oxide and 20 g of a glycerol fatty acid ester (Myvacet 9/08) were dissolved and/or dispersed in a mixture of 80% ethanol with water. The intermediately coated tablets obtained in Examples 21 and 22 were then coated with a resulting solution in coating pan.

### Example 25: Fluid-bed granulation

Hydroxypropyl cellulose (HPC Klucel EF) and magnesium oxide (Magnesia 27) were dispersed in the solution of rabeprazole sodium in ethanol. Obtained dispersion was sprayed onto granulated magnesium oxide particles in fluid bed granulating system to give granules. Dry granules, mannitol (Parteck M200), magnesium oxide (Magnesia 27), low-substituted hydroxypropyl cellulose (L-HPC LH-11) and magnesium stearate were mixed together and treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.50 |
| Magnesium oxide | 12.00 |
| Magnesium oxide, granulated | 48.00 |
| Hydroxypropyl Cellulose, Low-substituted | 27.25 |
| Hydroxypropyl Cellulose | 2.25 |
| Magnesium stearate | 3.00 |

### Example 26: Fluid-bed granulation

Hydroxypropyl cellulose (HPC Klucel EF) and magnesium oxide (Magnesia 27) were dispersed in the solution of rabeprazole sodium in ethanol. Obtained dispersion was sprayed onto granulated magnesium oxide particles and low-substituted hydroxypropyl cellulose (L-HPC LH-11) in fluid bed granulating system to give granules. Dry granules, mannitol (Parteck M200), magnesium oxide (Magnesia 27), additional low-substituted hydroxypropyl cellulose (L-HPC LH-11) and magnesium stearate were mixed together and compressed into drug containing tablet cores of the following composition each weighing 150.0 mg.

### Composition:

| Ingredient | mg/tablet core |
|---|---|
| Rabeprazole sodium | 20.00 |
| Mannitol | 37.50 |
| Magnesium oxide | 12.00 |
| Magnesium oxide, granulated | 48.00 |
| Hydroxypropyl Cellulose, Low-substituted | 27.25 |
| Hydroxypropyl Cellulose | 2.25 |
| Magnesium stearate | 3.00 |

### Example 27: Intermediate coating

40 g of ethylcellulose (Ethocel 20cP) was dissolved in 580 g of ethanol and 60 g of magnesium oxide (Magnesia 27) was dispersed in the obtained solution. The uncoated tablets obtained in Example 26 were intermediately coated with the above dispersion. Thus intermediately coated tablets with an average weight 152.3 mg were obtained. Tablet cores prepared according to Examples 25 were coated with the same procedure.

### Example 28: Enteric coating

200 g of hydroxypropyl methylcellulose phthalate, 10 g of titanium oxide, 20 g of talc, 2 g of red iron oxide and 20 g of a glycerol fatty acid ester (Myvacet 9/08) were dissolved and/or dispersed in a mixture of 80% ethanol with water. The intermediately coated tablets obtained in Example 27 were then coated with a resulting solution in coating pan. Thus enteric coated tablets with an average weight 166 mg were obtained.

### Example 29: Fluid-bed granulation

Rabeprazole sodium, hydroxypropyl cellulose (HPC Klucel EF) were dissolved in ethanol and magnesium oxide (Magnesia 27) was dispersed in the obtained solution. Obtained dispersion was sprayed onto granulated magnesium oxide particles and low-substituted hydroxypropyl cellulose (L-HPC LH-11) in fluid bed granulating system to give granules. Dry granules, mannitol (Parteck M200), magnesium oxide (Magnesia 27), additional low-substituted hydroxypropyl cellulose (L-HPC LH-11) and magnesium stearate were mixed together and treated with a tablet machine to thereby give uncoated tablets of the following composition each weighing 150.0 mg

### Composition:

| Ingredient | mg/tablet core |
|---|---|
| Rabeprazole sodium | 20.00 |
| Mannitol | 36.00 |
| Magnesium oxide | 12.00 |
| Magnesium oxide, granulated | 48.00 |
| Hydroxypropyl Cellulose, Low-substituted | 27.25 |
| Hydroxypropyl Cellulose | 2.25 |
| Magnesium stearate | 4.50 |

### Example 30: Wet granulation

Rabeprazole sodium, mannitol, magnesium oxide (Magnesia 27), hydroxypropyl cellulose (HPC Klucel EF), low-substituted hydroxypropyl cellulose (L-HPC LH-11) were sieved and mixed together. To the obtained mixture ethanol was added. The resulting mixture was granulated, dried and passed through an 18-mesh sieve to give dry granules (A), which were mixed with additional mannitol (Parteck M200), additional low-substituted hydroxypropyl cellulose (L-HPC LH-11) and magnesium stearate. The mixture was treated with a tablet machine to thereby give uncoated tablets of the following composition each weighing 150.0 mg

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 26.25 |
| Hydroxypropyl Cellulose | 4.50 |
| Magnesium stearate | 2.25 |

### Example 31: Wet granulation

Mannitol, magnesium oxide (Magnesia 27), hydroxypropyl cellulose (HPC Klucel EF), low-substituted hydroxypropyl cellulose (L-HPC LH-11) were sieved and mixed together. The obtained mixture was granulated with a dispersion obtained by dispersing a part of magnesium oxide in solution of rabeprazole sodium in ethanol. The resulting wet granulate was dried and passed through an 18-mesh sieve to give dry granules (A), which were mixed with additional mannitol (Parteck M200), additional low-substituted hydroxypropyl cellulose (L-HPC LH-11) and magnesium stearate. The mixture was treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 27.75 |
| Hydroxypropyl Cellulose | 3.00 |
| Magnesium stearate | 2.25 |

### Example 32: Wet granulation

Mannitol, magnesium oxide (Magnesia 27), hydroxypropyl cellulose (HPC Klucel EF), low-substituted hydroxypropyl cellulose (L-HPC LH-11) were sieved and mixed together. The obtained mixture was granulated with a dispersion obtained by dispersing a part of magnesium oxide in solution of rabeprazole sodium in ethanol. The resulting wet granulate was dried and passed through an 18-mesh sieve to give dry granules (A), which were mixed with additional mannitol (Parteck M200), additional low-substituted hydroxypropyl cellulose (L-HPC LH-11) and magnesium stearate. The mixture was treated with a tablet machine to thereby give uncoated tablets of the following composition each weighing 150.0 mg.

### Composition:

| Ingredient | mg/tablet core |
|---|---|
| Rabeprazole sodium | 20.00 |
| Mannitol | 37.00 |
| Magnesium oxide | 60.00 |
| Hydroxypropyl Cellulose, Low-substituted | 25.50 |
| Hydroxypropyl Cellulose | 5.25 |
| Magnesium stearate | 2.25 |

### Example 33: Intermediate coating

40 g of ethylcellulose (Ethocel 20cP) was dissolved in 580 g of ethanol and 60 g of magnesium oxide (Magnesia 27) was dispersed in the obtained solution. The uncoated tablets obtained in Example 29 were intermediately coated with the above dispersion. Thus intermediately coated tablets with an average weight 153 mg were obtained. Tablet cores prepared according to Examples 30-32 were coated with the same procedure.

### Example 34: Enteric coating

200 g of hydroxypropyl methylcellulose phthalate, 10 g of titanium oxide, 20 g of talc, 1 g of red iron oxide and 20 g of a glycerol fatty acid ester (Myvacet 9/08) were dissolved and/or dispersed in a mixture of 80% ethanol with water. The intermediately coated tablets obtained in Example 33 were then coated with a resulting solution in coating pan.

### Example 35: Stability test of rabeprazole sodium formulations

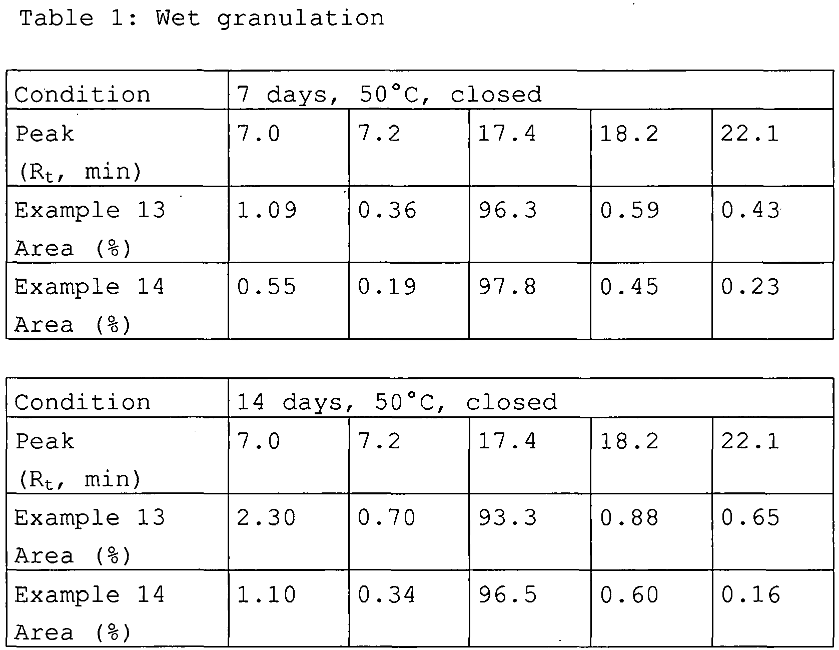

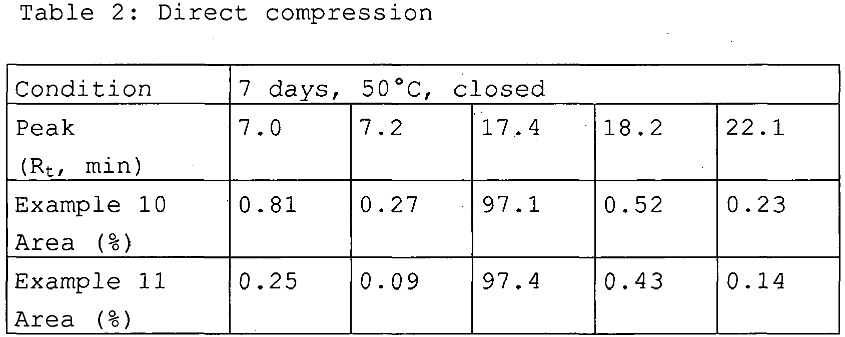

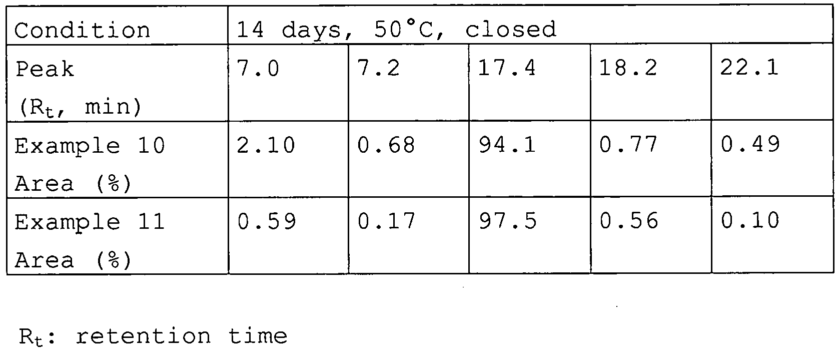

**Table 3: Rabeprazole and its major impurities**

| Rₜ [min] | Substance |
|---|---|
| 7.0 | 1-(1H-benzimidazol-2-yl)-2,3-dimethylpyridin-4(1H)-one |
| 7.2 | 4-methyl-3-oxo-5H-pyrido[1',2':4,5][1,2,4]thiadiazino[2,3-a]benzimidazole |
| 17.4 | rabeprazole |
| 18.2 | rabeprazole sulfone |
| 22.1 | rabeprazole sulfide |

**Table 4: Average particle size and specific surface area of different types of MgO**

| MgO type | granulated | Magnesia 22 | Magnesia 27 |
|---|---|---|---|
| Average particle size | 310µm | 16µm | 7µm |
| Specific surface area | 10m²/g | 7m²/g | 37m²/g |

In Example 13 Magnesia 22 was used, in Example 14 Magnesia 27, in Example 10 granulated MgO and in Example 11 mixture of granulated MgO and Magnesia 22 in ratio 1.4:1 was used. The average particle size of rabeprazole sodium was 25 µm.

As can be evaluated from tables 1, 2 and 4 smaller MgO particles or/and MgO particles with greater surface area give better stability of rabeprazole sodium.

The X-ray analysis of tablets prepared according to examples 19, 20, 25, 26, 29, 30, 31, 32 showed that rabeprazole sodium in final tablets was in an amorphous form.

## Claims

1. Crystalline form F of rabeprazole sodium **characterized by** an X-ray powder diffraction pattern having peaks at about 3.9, 5.6, 7.7, 11.6, 14.2 ± 0.2 degrees two-theta.

2. The crystalline form F of rabeprazole sodium according to claim 1, which is **characterized by** having peaks at about 3.9, 5.6, 7.7, 11.6, 14.2, 19.5, 24.6 ± 0.2 degrees two-theta.

3. The crystalline form F of rabeprazole sodium according to claim 1 or 2, which exhibits an X-ray powder diffraction pattern substantially the same as the pattern shown in Figure 9.

4. A process for the preparation of the crystalline form F of rabeprazole sodium according to any one of claims 1 to 3 comprising the steps of:
a) preparing a solution of rabeprazole sodium in a nitrile solvent with less than 10% molar excess of sodium cations in regard to rabeprazole;
b) crystallizing rabeprazole sodium from the solution by adding an antisolvent; and
c) isolating the resulting precipitate.

5. Process according to claim 4, wherein the nitrile solvent is acetonitrile.

6. Process according to claim 4 or 5, wherein the antisolvent is an ether.

7. Process according to claim 6, wherein the antisolvent is tert-butyl methyl ether or diisopropyl ether.

8. Process according to any one of claims 4 to 7, wherein the nitrile solvent and the antisolvent are used in a ratio of 5:1 to 1:5.

9. Process according to claim 8, wherein the nitrile solvent and the antisolvent are used in a ratio of 2:1 to 1:3.

10. Process according to claim 9, wherein the nitrile solvent and the antisolvent are used in a ratio of 1:1 to 1:2.

11. Process according to any one of claims 4 to 10, wherein the concentration of rabeprazole sodium at the beginning of crystallization is less than 0.3 g/ml.

12. Use of a crystalline form of rabeprazole sodium according to any one of claims 1 to 3 for the preparation of other crystalline or amorphous forms of rabeprazole or rabeprazole sodium.

13. Use of a crystalline form of rabeprazole sodium according to any one of claims 1 to 3 for the preparation of a pharmaceutical composition.

14. A pharmaceutical composition comprising the crystalline form of rabeprazole sodium according to any one of claims 1 or 3 in combination with at least one pharmaceutically acceptable carrier.

## Patentansprüche

1. Kristalline Form F von Rabeprazol-Natrium **gekennzeichnet durch** ein Röntgenpulverdiffraktogramm mit Peaks bei etwa 3,9, 5,6, 7,7, 11,6, 14,2 ± 0,2 Grad Zwei-Theta.

2. Kristalline Form F von Rabeprazol-Natrium nach Anspruch 1, **dadurch gekennzeichnet, dass** sie Peaks bei etwa 3,9, 5,6, 7,7, 11,6, 14,2, 19,5, 24,6 ± 0,2 Grad Zwei-Theta aufweist.

3. Kristalline Form F von Rabeprazol-Natrium nach Anspruch 1 oder 2, die ein Röntgenpulverdiffraktogramm aufweist, das dem in Abbildung 9 gezeigten Muster im Wesentlichen gleich ist.

4. Verfahren zur Herstellung der kristallinen Form F von Rabeprazol-Natrium nach einem der Ansprüche 1 bis 3, das die Schritte umfasst, dass
a) eine Lösung von Rabeprazol-Natrium in einem Nitril-Lösungsmittel mit weniger als 10% molarem Überschuss an Natrium-Kationen in Bezug auf Rabeprazol hergestellt wird,
b) Rabeprazol-Natrium aus der Lösung durch Zugabe eines Anti-Lösungsmittels kristallisiert wird, und
c) der erhaltene Niederschlag isoliert wird.

5. Verfahren nach Anspruch 4, bei dem das Nitril-Lösungsmittel Acetonitril ist.

6. Verfahren nach Anspruch 4 oder 5, bei dem das Anti-Lösungsmittel ein Ether ist.

7. Verfahren nach Anspruch 6, bei dem das Anti-Lösungsmittel tert-Butylmethylether oder Diisopropylether ist.

8. Verfahren nach einem der Ansprüche 4 bis 7, bei dem das Nitril-Lösungsmittel und das Anti-Lösungsmittel in einem Verhältnis von 5:1 bis 1:5 verwendet werden.

9. Verfahren nach Anspruch 8, bei dem das Nitril-Lösungsmittel und das Anti-Lösungsmittel in einem Verhältnis von 2:1 bis 1:3 verwendet werden.

10. Verfahren nach Anspruch 9, bei dem das Nitril-Lösungsmittel und das Anti-Lösungsmittel in einem Verhältnis von 1:1 bis 1:2 verwendet werden.

11. Verfahren nach einem der Ansprüche 4 bis 10, bei dem die Konzentration von Rabeprazol-Natrium am Anfang der Kristallisation weniger als 0,3 g/ml beträgt.

12. Verwendung einer kristallinen Form von Rabeprazol-Natrium nach einem der Ansprüche 1 bis 3 zur Herstellung von anderen kristallinen oder amorphen Formen von Rabeprazol oder Rabeprazol-Natrium.

13. Verwendung einer kristallinen Form von Rabeprazol-Natrium nach einem der Ansprüche 1 bis 3 zur Herstellung einer pharmazeutischen Zusammensetzung.

14. Pharmazeutische Zusammensetzung, die die kristalline Form von Rabeprazol-Natrium nach einem der Ansprüche 1 bis 3 in Kombination mit mindestens einem pharmazeutisch annehmbaren Träger umfasst.

## Revendications

1. Forme cristalline F de rabéprazole sodique, **caractérisée en ce que** son diagramme de diffraction des rayons X à l'état de poudre comporte des pics à des angles 2θ d'environ 3,9°, 5,6°, 7,7°, 11,6° et 14,2°, à ± 0,2° près.

2. Forme cristalline F de rabéprazole sodique, conforme à la revendication 1, **caractérisée en ce qu'**elle donne des pics à des angles 2θ d'environ 3,9°, 5,6°, 7,7°, 11,6°, 14,2°, 19,5° et 24,6°, à ± 0,2° près.

3. Forme cristalline F de rabéprazole sodique, conforme à la revendication 1 ou 2, dont le diagramme de diffraction des rayons X à l'état de poudre est sensiblement le même que le diagramme représenté sur la figure 9.

4. Procédé de préparation de la forme cristalline F de rabéprazole sodique conforme à l'une des revendications 1 à 3, qui comporte les étapes suivantes :
a) préparer une solution de rabéprazole sodique dans un solvant de type nitrile, dans laquelle les cations de sodium se trouvent, par rapport au rabéprazole, en excès de moins de 10 % en nombre de moles ;
b) faire cristalliser le rabéprazole sodique, à partir de cette solution, en y ajoutant un non-solvant ;
c) et isoler le précipité résultant.

5. Procédé conforme à la revendication 4, dans lequel le solvant de type nitrile est de l'acétonitrile.

6. Procédé conforme à la revendication 4 ou 5, dans lequel le non-solvant est un éther.

7. Procédé conforme à la revendication 6, dans lequel le non-solvant est du méthyl-tertiobutyl-éther ou de l'éther diisopropylique.

8. Procédé conforme à l'une des revendications 4 à 7, dans lequel le solvant de type nitrile et le non-solvant sont utilisés en un rapport valant de 5/1 à 1/5.

9. Procédé conforme à la revendication 8, dans lequel le solvant de type nitrile et le non-solvant sont utilisés en un rapport valant de 2/1 à 1/3.

10. Procédé conforme à la revendication 9, dans lequel le solvant de type nitrile et le non-solvant sont utilisés en un rapport valant de 1/1 à 1/2.

11. Procédé conforme à l'une des revendications 4 à 10, dans lequel la concentration de rabéprazole sodique au début de la cristallisation vaut moins de 0,3 g/mL.

12. Utilisation d'une forme cristalline de rabéprazole sodique conforme à l'une des revendications 1 à 3 pour la préparation d'autres formes, cristallines ou amorphes, de rabéprazole ou de rabéprazole sodique.

13. Utilisation d'une forme cristalline de rabéprazole sodique conforme à l'une des revendications 1 à 3 pour la préparation d'une composition pharmaceutique.

14. Composition pharmaceutique comprenant une forme cristalline de rabéprazole sodique conforme à l'une des revendications 1 à 3, en combinaison avec au moins un véhicule pharmacologiquement admissible.
